# EUROPÄISCHE PATENTSCHRIFT

(11) **EP 0 993 239 B1**
(45) Veröffentlichungstag und Bekanntmachung des Hinweises auf die Patenterteilung: **21.09.2005**
(21) Anmeldenummer: 99203176.5
(22) Anmeldetag: 29.09.1999
(51) Int. Cl.: H05G 1/46, A61B 6/06

(54) **Röntgeneinrichtung**
X-ray apparatus
Appareil à rayons X

(30) Priorität: 05.10.1998 DE 19845650
(43) Veröffentlichungstag der Anmeldung: 12.04.2000
(73) Patentinhaber: Philips Intellectual Property & Standards GmbH, 20099 Hamburg (DE); Koninklijke Philips Electronics N.V., 5621 BA Eindhoven (NL)
(72) Erfinder: Neumann, Kai, c/o Philips Corporate Intellectual, 52064 Aachen (DE); Spiess, Klaus, c/o Philips Corporate Intellectual, 52064 Aachen (DE); Biermann, Peter, c/o Philips Corporate Intellectua, 52064 Aachen (DE)
(74) Vertreter: Peters, Carl Heinrich

(56) Entgegenhaltungen:
- FR-A- 2 271 799
- US-A- 4 597 094
- US-A- 5 588 036

## Beschreibung

Die Erfindung bezieht sich auf eine Röntgeneinrichtung mit einem Röntgenaufnahmegerät und einem Röntgengenerator zur Speisung eines mit dem Röntgenaufnahmegerät zusammenwirkenden Röntgenstrahlers und mit einer mit dem Röntgenstrahler verbundenen Blendeneinheit mit einstellbarer Blendenöffnung zwecks Vorgabe eines Aufnahmefeldes auf einem Röntgen-Bildaufnehmer, wobei die Blendenöffnung einerseits durch eine von einem Steuersystem gesteuerte Antriebseinheit und andererseits durch Einstellmittel zum manuellen Einstellen der Blendenöffnung einstellbar ist.

Röntgeneinrichtungen dieser Art zum Anfertigen von Bucky-Aufnahmen sind bekannt. Als Bildaufnehmer werden dabei Film-Folienkombinationen unterschiedlichen Formats verwendet, die sich in Kassetten mit daran angepaßten Abmessungen befinden. Der Untersucher wählt dabei das für die nachfolgende Röntgenaufnahme erforderliche Kassettenformat aus und legt die Kassette in die Röntgeneinrichtung ein. Diese besitzt eine Meßeinrichtung zur Erfassung des Kassettenformates. Das Steuersystem verstellt dann in Abhängigkeit von dem gemessenen Kassettenformat die Blendenöffnung so, daß das Aufnahmefeld dem Kassettenformat bzw. dem Format des darin befindlichen Films entspricht. Ein manuelles Einstellen des Aufnahmefeldes ist bei derartigen Röntgeneinrichtungen nur erforderlich, wenn der Untersucher das Aufnahmefeld einengen will.

Neuerdings werden als Bildaufnehmer sogenannte "digitale" Röntgendetektoren verwendet, die eine Vielzahl (z.B. 2000x2000) von matrixförmig angeordneten, für Licht oder Röntgenstrahlen empfindlichen Detektorelementen enthalten, die von der Röntgenstrahlenintensität abhängige elektrische Signale erzeugen, die in der Röntgeneinrichtung verarbeitet werden. Die Röntgeneinrichtung kann verschiedene Aufnahmeeinheiten umfassen, z.B. einen Rasteraufnahmetisch für Röntgenaufnahmen am liegenden Patienten und/oder ein Rasterwandstativ für Röntgenaufnahmen am stehenden Patienten; jede dieser Einheiten enthält nur einen einzigen solchen digitalen Detektor, dessen Abmessungen deshalb dem größtmöglichen Aufnahmeformat (z.B. 43x43 cm) entsprechen müssen. Eine automatische Einstellung der Blendenöffnung auf das Format dieses Bildaufnehmers würde aber in den meisten Fällen eine teilweise erhebliche manuelle Einengung des Aufnahmefeldes erfordern, was die Bedienung eines solchen Gerätes erschweren würde.

Aufgabe der vorliegenden Erfindung ist es, die Bedienung einer Röntgeneinrichtung zu vereinfachen, deren Aufnahemeeinheit(en) einen Bildaufnehmer mit jeweils nur einem einzigen (maximalen) Format hat(haben). Diese Aufgabe wird ausgehend von einer Röntgeneinrichtung der eingangs genannten Art dadurch gelöst, daß eine mit dem Steuersystem zusammenwirkende Speicheranordnung vorgesehen ist, in der für eine Anzahl von Organen je ein Satz von Aufnahmeparametern gespeichert ist, daß jeder Satz neben Aufnahmeparametern für den Röntgengenerator einen Einstellwert zur Einstellung des Aufnahmefeldes enthält, und daß bei einer Vorgabe eines Organs der Einstellwert aufgerufen und das Aufnahmefeld über das Steuersystem und die Antriebseinheit entsprechend dem zu dem vorgegebenen Organ gehörigen Einstellwert eingestellt wird.

Die Verwendung einer Speicheranordnung, in der für verschiedene Organe je ein Satz von Aufnahmeparametern gespeichert ist, ist in der Röntgentechnik seit langem bekannt. Bei diesen sogenannten APR (Anatomically Programmed Radiography)-Verfahren werden im wesentlichen Aufnahmeparameter für den Röntgengenerator, z.B. die Spannung an der Röntgenröhre, der Strom durch die Röntgenröhre und die Aufnahmedauer organabhängig gespeichert und bei einer Auswahl dieses Organs aufgerufen und eingestellt.

Die Erfindung basiert auf der Erkenntnis, daß die Größe des Aufnahmefeldes mit dem Organ bzw. der Körperregion korreliert ist, die durch die nachfolgende Röntgenaufnahme abgebildet werden soll. Deshalb wird für jedes Organ zusätzlich die Größe des dafür erforderlichen Aufnahmefeldes gespeichert. Der gespeicherte Einstellwert wird bei einer Vorgabe dieses Organs aufgerufen und steuert über das Steuersystem und die Antriebseinheit die Blendeneinheit so, daß das vorgegebene Aufnahmefeld eingestellt wird. Der Untersucher muß danach das Aufnahmefeld - wenn überhaupt - nur geringfügig verändern.

Bei der manuellen Einstellung des Aufnahmefeldes befindet sich der Untersucher in der Nähe des - z.B. auf einem Patientenlagerungstisch gelagerten - Patienten. Die übrigen Einstellvorgänge hingegen, z.B. die Vorgabe eines Organs, die Auslösung einer Röntgenaufnahme usw. werden an einem Bedienpult bzw. einer Workstation vorgenommen, die sich in einem anderen Raum als der Patient befindet. Die Ausgestaltung nach Anspruch 2 verhindert dabei, daß die für die Aufnahme des betreffenden Organs manuell vorgegebene Einstellung durch einen für dieses Organ gespeicherten Einstellwert überschrieben und damit wieder rückgängig gemacht wird. Das entsprechend programmierte Steuersystem setzt sich dabei zweckmäßigerweise aus einem Blenden-Controller zusammen, der für die Steuerung der Antriebseinheit und der Blendeneinheit zuständig ist, sowie aus der Workstation, die alle Komponenten der Röntgeneinrichtung und den gesamten Aufnahmeablauf steuert.

Die weitere Ausgestaltung nach Anspruch 3 ermöglicht allerdings dann einen Übergang von der manuellen Einstellung auf die gespeicherten Einstellwerte, wenn nach der manuellen Einstellung eine Röntgenaufnahme oder ein Patientenwechsel erfolgt ist.

Bucky-Aufnahmen oder Aufnahmen am Wandstativ werden im allgemeinen mit einem bestimmten Abstand zwischen Röntgenstrahler und Bildaufnehmer ausgeführt, z.B. 1,15 m. In diesem Fall entspricht jedes Aufnahmefeld einer bestimmten Blendenöffnung. Vielfach ist es aber erwünscht, den Abstand zwischen Röntgenstrahler und Bildaufnehmer zu vergrößern oder zu verkleinern, und die Weiterbildung nach Anspruch 4 bewirkt nun, daß bei einer Veränderung des genannten Abstandes auch die Blendenöffnung so verstellt wird, daß sich das gewünschte Aufnahmefeld in der Ebene des Bildaufnehmers ergibt.

Anspruch 5 gibt an, bei welcher Art von Bildaufnehmern die Erfindung anwendbar ist. Grundsätzlich ist die Erfindung aber bei allen Aufnahmeeinheiten anwendbar, die lediglich über ein einziges Format des Bildaufnehmers verfügen.

Die Erfindung wird nachstehend anhand der Zeichnungen näher erläutert. Es zeigen:
- Fig. 1: eine erfindungsgemäße Röntgeneinrichtung und
- Fig. 2A-2E: Ablaufdiagramme für die Steuerung der Blendeneinheit.

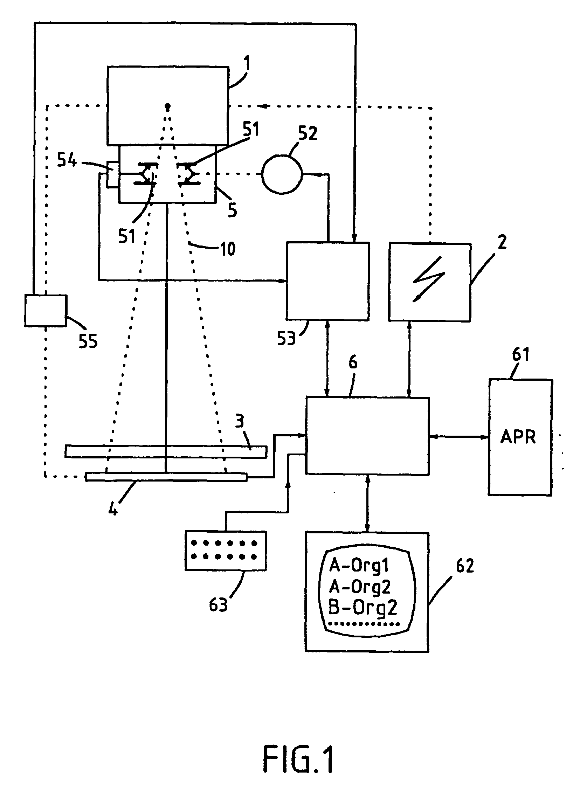

Die in Fig. 1 dargestellte Röntgeneinrichtung umfaßt einen Röntgenstrahler 1, der von einem Röntgengenerator 2 gesteuert wird. Der Röntgenstrahler ist an einem nicht näher dargestellten Stativ befestigt und kann daran zumindest in vertikaler Richtung verschoben werden. Außerdem umfaßt die Röntgeneinrichtung einen Patientenlagerungstisch, der durch eine Tischplatte 3 symbolisch angedeutet ist, unterhalb der sich ein digitaler Bildaufnehmer 4 mit matrixförmigen Detektorelementen befindet. Zusätzlich zu dem Patientenlagerungstisch - oder auch an seiner statt - kann ein nicht näher dargestelltes Rasterwandstativ mit einem derartigen Bildaufnehmer vorgesehen sein.

Die Größe des Aufnahmefeldes, das auf dem Bildaufnehmer 4 von dem Röntgenstrahler 1 bestrahlt wird, wird durch eine Blendeneinheit 5 eingestellt. Die Blendenöffnung, d.h. der Öffnungswinkel des gestrichelt angedeuteten Strahlenbündels in der Zeichenebene, wird durch einen ersten Satz von Kollimatoren 51 beispielsweise aus Blei bestimmt, deren Kanten senkrecht zur Zeichenebene der Fig. 1 verlaufen. Außerdem ist ein zweiter Satz von Kollimatoren vorgesehen, in Fig. 1 aber nicht dargestellt, der zur Zeichenebene parallele Blendenkanten aufweist und den Öffnungswinkel des Strahlenbündels 10 senkrecht zur Zeichenebene bestimmt. Die Blendenöffnung kann durch einen Motor 52 verstellt werden, der sich - anders als in der Zeichnung dargestellt - in der Blendeneinheit 5 befindet. Der Motor 52 wird von einem Blenden-Controller 53 gesteuert, die mit einer Workstation 6 zusammenarbeitet.

Außerdem kann die Blendenöffnung mit einem Einstellglied 54 manuell vom Untersucher eingestellt werden. Die Betätigung des Einstellgliedes 54 wird vom Blenden-Controller 53 registriert. Die Größe des eingestellten Aufnahmefeldes kann vor einer Aufnahme vom Untersucher mit Hilfe eines in der Blendeneinheit 5 untergebrachten, nicht näher dargestellten Lichtvisiers überprüft werden, das ein Lichtbündel erzeugt, das durch die Kollimatoren 51 usw. in gleicher Weise begrenzt wird wie bei der Aufnahme das Röntgenstrahlenbündel 10. Der Abstand zwischen dem Röntgenstrahler und dem Bildaufnehmer wird von einer Meßeinrichtung 55 gemessen, und der Meßwert wird dem Blenden-Controller 53 übermittelt.

Die Workstation 6 steuert u.a. den Blenden-Controller 53 und den Röntgengenerator 2. Sie kann dabei auf eine Speicheranordnung 61 zugreifen, in der in Form einer Datenbank für eine Vielzahl von Organen je ein Datensatz gespeichert ist. Jeder Datensatz enthält die für das jeweilige Organ im Normalfall optimalen Aufnahmepararneter, darunter auch einen Wert für die Größe des einzustellenden Aufnahmefeldes. Dieser Einstellwert kann dem Blenden-Controller 53 zwecks Einstellung des Aufnahmefeldes unter Berücksichtigung des gemessenen Abstandes zwischen Röntgenstrahler und Bildaufnehmer vorgegeben werden.

Die Workstation kann darüber hinaus aus den Signalen des Röntgenbildaufnehmers 4 eine Röntgenaufnahme rekonstruieren und diese auf einem Monitor 62 wiedergeben. Andererseits kann die Workstation auf dem Monitor eine Patienten- und Aufnahmeliste vorgeben, die neben dem Namen eines Patienten die Organe bzw. Körperregionen enthält, die für diesen Patienten durch eine Röntgenaufnahme dargestellt werden soll. Diese Liste kann der Workstation 6 beispielsweise über eine sogenannte RIS-Verbindung (Radiology Information System) vorgegeben werden. Außerdem ist eine Eingabeeinheit 63 vorgesehen, z.B. eine Tastatur- und/oder eine Touchscreen-Einheit,um mit der Workstation 6 zu kommunizieren. Der Patientenlagerungstisch 3 (ggf. auch das oben erwähnte Rasterwandstativ) mit dem Röntgenstrahler 1 und der Blendeneinheit 5 befinden sich in einem anderen Raum als die Komponenten 6, 61, 62 und 63. Bei jeder Röntgenaufnahme ist der Untersucher in beiden Räumen tätig: bei der Einstellung der Aufnahmeparameter durch Vorgabe des Organs sowie bei der Auslösung einer Röntgenaufnahme in dem einen Raum und bei der Positionierung des Patienten und ggf. der manuellen Einstellung der Blendeneinheit in dem anderen Raum.

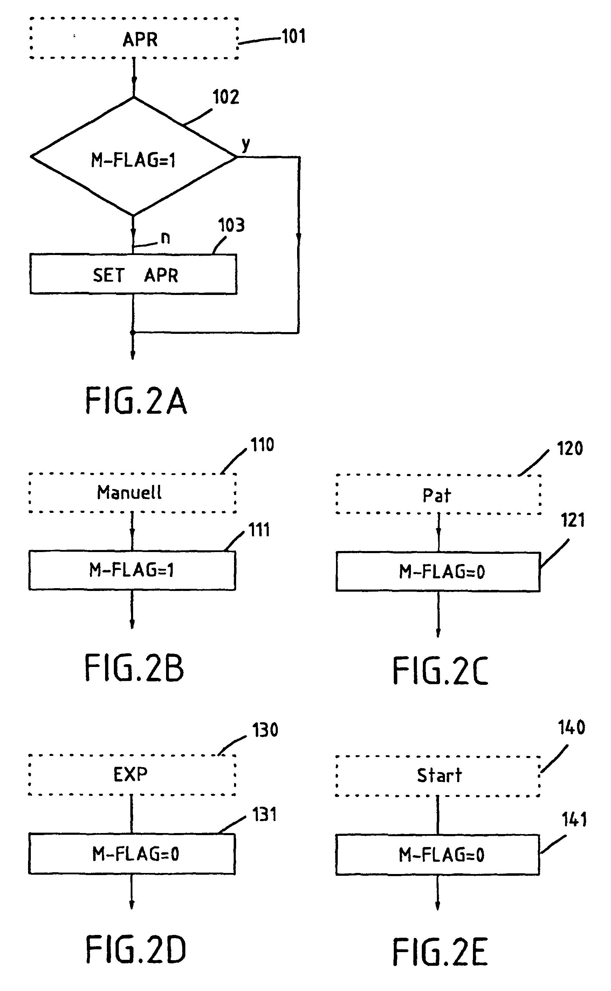

In den Fig. 2A bis 2E sind Teile von Ablaufdiagrammen dargestellt, die die Einstellung des Aufnahmefeldes bestimmen. Gemäß Fig. 2A erfolgt nach der Vorgabe eines Organs bzw. dem Aufruf von APR-Daten im Schritt 101 eine Abfrage, ob ein bestimmtes Flag gesetzt ist (M-FLAG= 1) oder nicht (Schritt 102). Wenn das Flag nicht gesetzt ist, erzeugt die Workstation im Schritt 103 den Befehl für den Blenden-Controller 53, die Größe des Aufnahmefeldes unter Berücksichtigung des Abstandes zwischen Röntgenstrahler 1 und Bildaufnehmer 4 entsprechend dem für das aufzunehmende Organ gespeicherten Einstellwert einzustellen. Ist hingegen das Flag gesetzt, bleibt die Einstellung unverändert. Wie in den Fig. 2B.....2E dargestellt, kann das Flag in Abhängigkeit von vier verschiedenen Ereignissen gesetzt bzw. zurückgesetzt werden:

Gemäß Fig. 2B wird im Falle einer manuellen Einstellung der Blendenöffnung mittels des Einstellgliedes 54 (Block 110) das Flag im Schritt 111 gesetzt (M-Flag=1). Wenn hingegen ein Patientenwechsel erfolgt (Block 120), wird im Schritt 121 das Flag zurückgesetzt (M-FLAG=0 - Fig. 2C). Das gleiche passiert im Schritt 131 gemäß Fig. 2D nach Auslösung einer Röntgenaufnahme (Block 130) oder im Schritt 141 bei einem (Neu-) Start des Systems 140.

Im folgenden wird erläutert, wie die Röntgeneinheit arbeitet, wenn der Untersucher eine auf dem Monitor 62 vorgegebene Patienten- und Aufnahmeliste abarbeitet. Dabei sei angenommen, daß die Liste nacheinander als erste Aufnahme eine Aufnahme des Patienten A mit dem Organ 1 (z.B. eine laterale Lungenaufnahme) anfordert und als zweite Aufnahme eine weitere Aufnahme des Patienten A für ein zweites Organ, z.B. Lunge p.a. (obwohl in beiden Fällen die Lunge aufgenommen werden soll, werden die p.a. und die laterale Aufnahme dieses Organs wie verschiedene Organe mit einem unterschiedlichen Satz von Aufnahmeparametern behandelt). Als dritte Aufnahme möge die Patienten- und Aufnahmeliste eine Aufnahme des gleichen Organs (Lunge p.a.) bei einem anderen Patienten B vorschreiben.

Bei der ersten Aufnahme lagert der Untersucher den Patienten A auf dem Patientenlagerungstisch (oder er positioniert ihn vor dem erwähnten Wandstativ) und stellt an der Blendeneinheit mit dem Einstellglied 54 die Größe des Aufnahmefeldes ein, wodurch das Flag gesetzt wird,. wie in Fig. 2B dargestellt. Der Untersucher geht dann in den Raum mit der Workstation und stellt die Röntgeneinrichtung auf die erste Aufnahme ein, d.h. es wird für die Aufnahme der Patient A mit dem Organ 1 vorgegeben, wodurch ein Einstellwert für das Aufnahmefeld aufgerufen wird. Da das Flag jedoch gesetzt ist, wird gemäß dem in Fig. 2A dargestellten Ablauf das Aufnahmefeld nicht auf diesen aufgerufenen Einstellwert eingestellt. Der Untersucher löst dann die erste Röntgenaufnahme aus, was zur Folge hat, daß gemäß Fig. 2D das Flag gemäß dem Schritt 131 zurückgesetzt wird.

Der Patientenlagerungstisch kann ggf so ausgebildet sein, daß Röntgenstrahler 1 und Bildaufnehmer 4 gleichzeitig und gegensinnig zueinander verschoben und Schichtaufnahmen erstellt werden können. In diesem Schichtaufnahme-Modus wird meist eine Serie von Schichten in unterschiedlicher Tiefe aufgenommen, und deshalb darf in diesem Modus eine (Schicht-)Aufnahme nicht zu der oben beschriebenen Zurücksetzung des Flags führen.

Wenn der Untersucher daher an der Workstation die zweite Aufnahme vorgibt, wird gemäß Fig. 2A der Einstellwert für das zweite Organ (Lunge pa) aufgerufen und über den Blenden-Controller 53 und durch den Motor 52 eingestellt, so daß die von der vorigen Aufnahme vorhandene manuelle Einstellung überschrieben wird. Der Untersucher lagert dann den Patienten A so, daß die zweite Aufnahme ausgeführt werden kann. Wenn er dabei das Einstellglied 54 betätigt, wird die Voreinstellung der Blendeneinheit durch den aus dem Speicher 61 für Lunge p.a. aufgerufenen Einstellwert entsprechend geändert, was ein erneutes Setzen des Flags zur Folge hat.

Wenn nach dieser zweiten Aufnahme an der Workstation 3 die dritte Aufnahme (Patient B) in der Liste vorgegeben wird, wird das Flag wegen des Patirentenwechssels zurückgesetzt (Schritt 121), selbst wenn dasselbe Organ aufzunehmen ist wie bei der vorangegangenen Aufnahme. Die bei der zweiten Aufnahme vorgenommenen manuellen Einstellungen werden deshalb entsprechend dem für das bei der dritte aufzunehmende Organ (Lunge pa) vorgegebenen Einstellwert überschrieben. Diese Voreinstellung kann der Untersucher - falls erforderlich - wiederum mit dem Einstellglied 54 verändern.

Der beschriebene Ablauf zeigt, daß die Röntgeneinrichtung dem Untersucher ein Höchstmaß an Ergonomie bei gleichzeitig hoher Flexibilität der Einstellung bietet.

## Patentansprüche

1. Röntgeneinrichtung mit einem Röntgenaufnahmegerät (3) und einem Röntgengenerator (2) zur Speisung eines mit dem Röntgenaufnahmegerät zusammenwirkenden Röntgenstrahlers (1) und mit einer mit dem Röntgenstrahler (1) verbundenen Blendeneinheit (5) mit einstellbarer Blendenöffnung zwecks Vorgabe eines Aufnahmefeldes auf einem Röntgen-Bildaufnehmer (4), wobei die Blendenöffnung einerseits durch eine von einem Steuersystem (6, 53) gesteuerte Antriebseinheit (52) und andererseits durch Einstellmittel (54) zum manuellen Einstellen der Blendenöffnung einstellbar ist, **dadurch gekennzeichnet, daß** eine mit dem Steuersystem (6, 53) zusammenwirkende Speicheranordnung (61) vorgesehen ist, in der für eine Anzahl von Organen je ein Satz von Aufnahmeparametern gespeichert ist, daß jeder Satz neben Aufnahmeparametern für den Röntgengenerator einen Einstellwert zur Einstellung des Aufnahmefeldes enthält und daß bei einer Vorgabe eines Organs der Einstellwert aufgerufen und das Aufnahmefeld über das Steuersystem und die Antriebseinheit entsprechend dem zu dem vorgegebenen Organ gehörigen Einstellwert eingestellt wird.

2. Röntgeneinrichtung nach Anspruch 1,
**dadurch gekennzeichnet, daß** das Steuersystem (6, 53) so programmiert ist, daß nach einer Betätigung der Einstellmittel (54)die manuelle Einstellung des Aufnahmefeldes unabhängig von einem davor bzw. danach aufgerufenen Einstellwert erfolgt bzw. erhalten bleibt.

3. Röntgeneinrichtung nach Anspruch 2
**dadurch gekennzeichnet, daß** das Steuersystem (6, 53) so programmiert ist, daß ein durch Betätigung der Einstellmittel (54) eingestelltes Aufnahmefeld nach einer Röntgenaufnahme oder einem Wechsel eines zu untersuchenden Patienten entsprechend dem jeweils aufgerufenen Einstellwert eingestellt wird.

4. Röntgeneinrichtung nach Anspruch 1
**dadurch gekennzeichnet, daß** der Abstand zwischen dem Röntgenstrahler (1) und dem Röntgen-Bildaufnehmer (4) einstellbar ist, daß Mittel (55) zum Messen des Abstandes vorgesehen ist, und daß das Steuersystem (6, 53) so programmiert ist, daß die Blendenöffnung in Abhängigkeit von dem gemessenen Abstand einen solchen Wert hat, daß das Aufnahmefeld auf dem Bildaufnehmer (4) seinen vorgegebenen Wert annimmt.

5. Röntgeneinrichtung nach Anspruch 1
**dadurch gekennzeichnet, daß** als Röntgen-Bildaufnehmer (4) ein ebener Detektor mit matrixförmig angeordneten, licht - oder röntgenstrahlenempfindlichen Detektorelementen vorgesehen ist.

## Claims

1. An X-ray device which includes an X-ray imaging apparatus (3) and an X-ray generator (2) for powering an X-ray source (1) which co-operates with the X-ray imaging apparatus and also includes a diaphragm unit (5) which is connected to the X-ray source (1) and includes an adjustable diaphragm aperture in order to preset an exposure field on an X-ray image detection device (4), the diaphragm aperture being adjustable on the one hand by a drive unit (52) which is controlled by a control system (6, 53) and, on the other hand, by adjusting means (54) for manual adjustment of the diaphragm aperture, **characterized in that** a storage device (61) co-operating with the control system (6, 53) is provided in which device a respective set of exposure parameters is stored for each of a number of organs, that each set includes, in addition to the exposure parameters for the X-ray generator; an adjustment value for adjusting the exposure field, and that, when an organ is selected, the adjustment value is fetched and the exposure field is adjusted, by way of the control system and the drive unit, in conformity with the adjustment value associated with the selected organ.

2. An X-ray device as claimed in claim 1, **characterized in that** the control system (6, 53) is programmed in such a manner that, after actuation of the adjustment means (54), the manual adjustment of the exposure field is carried out or preserved independently of an adjustment value fetched before or after that.

3. An X-ray device as claimed in claim 2, **characterized in that** the control system (6, 53) is programmed in such a manner that an exposure field adjusted by actuation of the adjusting means (54) is adjusted in conformity with the respectgive adjustment value fetched subsequent to an X-ray exposure or a change of patient to be examined.

4. An X-ray device as claimed in claim 1, **characterized in that** the distance between the X-ray source (1) and the X-ray image detection device (4) is adjustable, that means (55) are provided for measuring this distance, and that the control system (6, 53) is programmed in such a manner that in dependence on the measured distance the diaphragm aperture has a value such that the size of the exposure field on the image detection device (4) assumes its preset value.

5. An X-ray device as claimed in claim 1, **characterized in that** use is made of an X-ray image detection device (4) in the form of a flat detector with light-sensitive or X-ray-sensitive detector elements which are arranged in the form of a matrix.

## Revendications

1. Appareil à rayons X avec un appareil radiographique (3) et un générateur de rayons X (2) pour l'alimentation d'un générateur de rayons X (1) coopérant avec l'appareil radiographique et avec une unité à diaphragme (5) reliée au générateur de rayons X (1) et dotée d'une ouverture de diaphragme réglable en vue de la détermination préalable d'un champ de prise de vue sur un appareil à prise de vue radiographique (4), l'ouverture du diaphragme étant réglable, d'une part, par une unité d'entraînement (52) commandée par un système de commande (6, 53) et, d'autre part, par des moyens de réglage (54) pour le réglage manuel de l'ouverture du diaphragme,
**caractérisé en ce qu'**il est prévu un dispositif de mémoire (61) coopérant avec le système de commande (6, 53) dans lequel, pour un nombre d'organes, il est respectivement enregistré un jeu de paramètres de prise de vue, que chaque jeu contient, outre les paramètres de prise de vue pour le générateur de rayons X, un paramètre de réglage pour le réglage du champ de prise de vue et qu'en cas de détermination préalable d'un organe, la valeur de réglage est appelée et le champ de prise de vue est réglé par l'intermédiaire du système de commande et de l'unité d'entraînement en fonction du paramètre de réglage correspondant à l'organe préalablement déterminé.

2. Appareil à rayons X selon la revendication 1,
**caractérisé en ce que** le système de commande (6, 53) est programmé de telle sorte qu'après un actionnement des moyens de réglage (54), le réglage manuel du champ de prise de vue soit effectué ou maintenu indépendamment d'un paramètre de réglage consulté préalablement ou ultérieurement.

3. Appareil à rayons X selon la revendication 2,
**caractérisé en ce que** le système de commande (6, 53) est programmé de telle sorte qu'un champ de prise de vue réglé par l'actionnement des moyens de réglage (54) soit réglé après une radiographie ou un changement d'un patient à examiner en fonction du paramètre de réglage respectivement appelé.

4. Appareil à rayons X selon la revendication 1,
**caractérisé en ce que** l'intervalle entre le générateur de rayons X (1) et le dispositif de prise de vue radiographique (4) est réglable de telle sorte qu'il soit prévu des moyens (55) pour la mesure de l'intervalle et que le système de commande (6, 53) soit programmé de telle sorte que l'ouverture du diaphragme en fonction de l'intervalle mesuré ait une valeur telle que le champ de prise de vue adopte une valeur préalablement déterminée sur le dispositif de prise de vue (4).

5. Appareil à rayons X selon la revendication 1,
**caractérisé en ce qu'**un détecteur plat avec des éléments détecteurs disposés sous forme matricielle et sensibles à la lumière et aux rayons X est prévu comme dispositif de prise de vue radiographique (4).
